# EUROPEAN PATENT SPECIFICATION

(11) **EP 3 747 401 B1**
(45) Date of publication and mention of the grant of the patent: **06.11.2024**
(21) Application number: 19382589.0
(22) Date of filing: 11.07.2019
(51) Int. Cl.: A61F 2/16, G02C 7/02

(54) **INTRAOCULAR LENS AND METHODS FOR OPTIMIZATION OF DEPTH OF FOCUS AND THE IMAGE QUALITY IN THE PERIPHERY OF THE VISUAL FIELD**
INTRAOKULARLINSE UND VERFAHREN ZUR OPTIMIERUNG DER FOKUSTIEFE UND DER BILDQUALITÄT IN DER PERIPHERIE DES SICHTFELDES
LENTILLE INTRAOCULAIRE ET PROCÉDÉS D'OPTIMISATION DE LA PROFONDEUR DE CHAMP ET DE LA QUALITÉ D'IMAGE DANS LA PÉRIPHÉRIE DU CHAMP VISUEL

(30) Priority: 07.06.2019 ES 201930521
(43) Date of publication of application: 09.12.2020
(73) Proprietor: VOPTICA S.L., 30111 Murcia (ES)
(72) Inventor: ARTAL SORIANO, Pablo, 30111 Murcia (ES); GINIS, Harilaos, 30111 Murcia (ES); BARRERA VERDEJO, Maria, 30111 Murcia (ES); VILLEGAS RUIZ, Eloy, 30111 Murcia (ES); PRIETO CORRALES, Pedro, 30111 Murcia (ES); TABERNERO DE PAZ, Juan, 30111 Murcia (ES)
(74) Representative: Elzaburu S.L.P.

(56) References cited:
- EP-A1- 2 631 891
- WO-A1-2016/040331
- WO-A2-2005/077300
- WO-A2-2006/053216

## Description

### Field of invention

The present invention refers to an intraocular lens used in ophthalmology, specifically for cataract surgery. Additionally, it refers to a method for selecting an intraocular lens to optimize depth of focus through customized asphericity and the image quality in the periphery of the visual field that uses said intraocular lenses.

### Background of the invention

The crystalline lens is a complex structure, whose function has been a topic of study for centuries. It presents a gradient in its refractive index ranging from about 1.390 at the lens surface to about 1.409 at its core. This gradient is achieved thanks to a variable concentration of proteins in the cytoplasm of lens fiber cells. Despite the high presence of proteins, the healthy crystalline lens shows a high transparency due to the compact distribution of proteins. In addition, the crystalline lens has aspherical surfaces, which reduces spherical aberration in the eye. On the other hand, the design of the human eye also allows the optimization of the formation of the image at the periphery in terms of aberrations and field curvature with respect to the shape of the retina.

Cataracts are a pathological condition in which the natural crystalline lens has lost its transparency and the formation of image in the retina is deteriorated mainly due to the scattering of light. In most cases, to solve this problem, cataract surgery is performed by which the damaged lens is removed from the eye and a specific implant (an intraocular lens IOL) is placed to substitute the extracted crystalline lens.

Intraocular lenses have been used in ophthalmology for more than half a century. Since then, IOL implantation has become the most common and successful surgery, not only in the field of ophthalmology, but in the field of medicine in general. The implant is chosen to provide the refractive power needed to optimize the focal point in the fovea, the highest resolution area of the retina, thus replacing the patient's crystalline lens. The central visual field of the pseudo-phakic eye (with the lens replaced by an IOL) is characterized by good image quality, almost entirely limited by the post-operative sphero-cylindrical error, or even in some cases and depending on the type of implant used, by the induction of spherical aberration.

In most cases, the optical quality at the periphery of the visual field is not considered to be one of the most important points in the design of an IOL, since, for major eccentricities in the retina, the number of photoreceptor cells decreases and neuronal convergence increases. Due to these physiological limitations of the retina, IOL manufacturers have designed ocular implants focusing solely on image quality in the central area of the fovea, regardless of what occurs in the far periphery of the visual field. While the natural crystalline lens has properties adapted to the shape of the retina, IOLs do not contemplate this phenomenon at all. However, there are specific applications where the quality of peripheral vision can have a great impact on the quality of vision, such as detecting the direction of movement of visual patterns or even simpler activities such as detecting small peripheral objects. It is possible that the mismatch between the field curvature provided by conventional IOLs and the shape of the retina produces very low contrast and quality images that are related to accidents due to the non-detection of obstacles in various areas of the visual field.

Another common phenomenon that occurs in the distant periphery in pseudo-phakic patients, of a rather unclear etiology, is the presence of dark shadows, and is referred to as negative dysphotopsy.

Although the peripheral behaviour of pseudophakic eyes has not yet been studied in depth, there is some evidence that IOLs are not capable of reproducing the peripheral image quality that the natural crystalline lens does bring to human vision (see B. Jaeken, S. Mirabet, J. M. Marín, P. Artal, "Comparison of the Optical Image Quality in the Periphery of Phakic and Pseudophakic Eyes", Invest Ophthalmol Vis Sci, 54, 3594- 3599, (2013)). Intraocular lenses are specifically designed to facilitate surgical techniques (such as ease of implantation), with the goal of regaining emmetropia and prioritizing central vision.

In most cases, current intraocular lenses induce problems such as increased astigmatism and other aberrations on the periphery of the pseudophakic eye's visual field. Although in principle these phenomena may be imperceptible directly by the patient (as would be the presence of blur in the central vision), there is evidence that they play a role in the patient's spatial orientation and safety.

On the other hand, there are currently several IOLs with different asphericity values, both to compensate for corneal asphericity and to extend the depth of focus and allow acceptable vision at various distances. A problem with these IOLs is that they provide a unique asphericity value. However, each patient has a cornea with a different value of spherical aberration to be considered. In addition, each patient has a different sensitivity to the final net amount of spherical aberration after implantation of the IOL.

On the other hand, objective and visual measurements on each patient can be made using adaptive optics visual simulation technology (see P. A. Piers, E. J. Fernández, S. Manzanera, S. Norrby, P. Artal, "Adaptive optics simulation of intraocular lenses with modified spherical aberration" Invest. Ophthalmol. Vis. Sci. 45, 4601-4610 (2004)).

Many types of IOLs have already been implemented in the patent and scientific literature. For example, US4504982A (Burk) describes a generic aspherical lens for intraocular implantation that has a biconvex shape. However, in its design there is no provision for peripheral vision.

CA2587432C (Norrby) describes a method for selecting an intraocular lens, involving only optical and anatomical measurements that are subsequently used to calculate the power and spherical aberration of the required IOL. This invention does not consider possible arrangements for peripheral image quality and, furthermore, does not include the preoperative subjective (psychophysical) evaluation of patients.

AU2007247491 B2 (Gerlach) describes a method for designing an intraocular lens with a given asphericity to compensate for corneal aberrations, considering the possible eccentricity of the pupil. The same principles are described in a later document by the same inventors (US8235525B2 (Lesage)). The above mentioned invention does not include provisions for peripheral image quality.

Document WO2005098518A1 (Piers) describes a hybrid lens with at least one diffractive element that is used to control the total longitudinal chromatic aberration of the eye.

CA2431470C (Piers) presents an IOL design that compensates for the spherical aberration of the cornea without mentioning its customization.

US10010407B2 (Rosen), describes an IOL design to improve wavefront specific aberrations such as coma or astigmatism for the peripheral visual field for angles up to 40 degrees, without reference to peripheral image quality and distortion.

WO 2016/040331 A1, referred to "Ophthalmic implants with extended depth of field and enhanced distance visual acuity", discloses a lens configured for implantation into an eye of a human that can include an optic including transparent material. The optic can have an anterior surface and a posterior surface. The anterior surface can be convex and the posterior surface can be concave such that the optic is meniscus shaped. Each of the convex anterior surface and the concave posterior surface can have a surface vertex. The optic can have an optical axis through the surface vertices and a thickness along the optical axis that is between about 100-700 micrometers. The lens can also include haptic portions disposed about the optic to affix the optic in the eye when implanted therein. The anterior and posterior surfaces can include aspheric surfaces.

EP 2631891 A1 discloses a method and system for simulating/emulating vision using intraocular lenses or devices prior to surgery.

There is currently a need in the intraocular lens market for an implant that provides an optimization of field curvature to fit it to the shape of the retina to optimize image quality in the far periphery of the visual field. In addition, it is desirable that the lenses have a range of spherical net aberration values to be customized to increase the depth of field customized to each patient.

### Summary of the invention

The object of the present invention is to provide an intraocular lens that solves the aforementioned inconveniences.

The invention provides an intraocular lens comprising a central part and a peripheral part, the central part being the optical part and the peripheral part comprising haptics, in which the central part comprises:
- an aspherical concave anterior surface, which is the surface closest to the iris of the eye once the lens has been implanted in the eye, and
- an aspherical convex posterior surface, which is the surface closest to the retina of the eye once the lens has been implanted in the eye,
such that:
- the radius of curvature of the posterior surface of the central part is smaller than the radius of curvature of the anterior surface of the central part, with a ratio between radii of between 2 and 6, and
- the haptics are arranged at an angle of between 0° and 10° with respect to a plane passing through the joints between the central part and the peripheral part and which is perpendicular to the optical axis of the eye in which the lens is intended to be implanted.

This intraocular lens, when implanted in the posterior chamber of the eye, simulates the natural imaging formation properties of the crystalline lens, especially in the far periphery of the visual field, optimizing the field curvature of the implanted eye to adapt it appropriately to the shape of the retina.

This intraocular lens results in an improved image formation in terms of sharpness, peripheral refraction and geometric distortion.

The invention also provides methods for optimizing depth of focus and image quality in the periphery of the visual field using said intraocular lens, according to claim 8.

Other characteristics and advantages of the present invention will be derived from the detailed description that follows from an illustrative embodiment of its object in relation to the accompanying figures.

### Brief description of the drawings

The object of the present invention will be illustrated below in a non-limiting way, making reference to the drawings that are accompanied, in which:
Figure 1 shows a section of a human eye and the focusing of objects in the center of the visual field and on the periphery of the visual field.
Figure 2 shows a section of a human eye with a prior art intraocular lens implanted in the posterior chamber of the eye, and the focusing of objects in the center of the visual field and on the periphery of the visual field.
Figure 3 shows a section of a human eye with an intraocular lens of the invention implanted in the posterior chamber of the eye, and the focusing of objects in the center of the visual field and on the periphery of the visual field.
Figures 4a and 4b show two embodiments of intraocular lenses of the invention.
Figure 5 schematically shows a visual simulator of adaptive optics and its use in the method to optimize depth of focus and image quality at the periphery of the visual field of invention.

### Detailed description of the invention

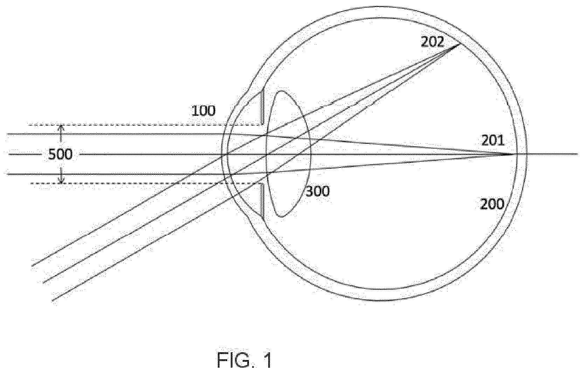

Figure 1 shows a section of the human eye. The light 500 entering through the pupil is focused by the cornea 100 and the lens of the crystalline 300 on the retina 200. Objects in the center of the visual field are focused on the central part 201 of the retina, while objects on the periphery of the visual field are focused on a peripheral point 202 of the retina.

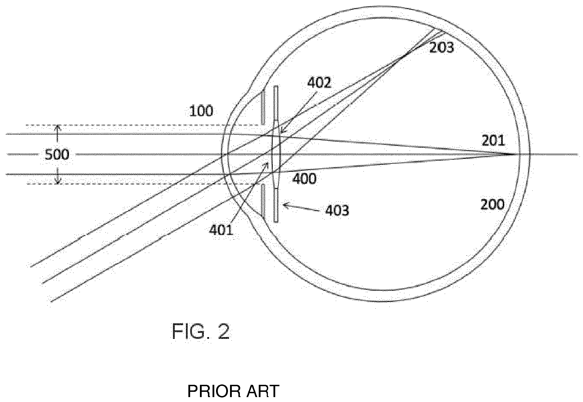

Figure 2 shows an eye with a prior art intraocular lens 400 implanted in the posterior chamber of the eye. The light 500 entering through the pupil is focused by the cornea 100 on the retina 200. The intraocular lens 400 has an anterior surface 401, a posterior surface 402 and haptics 403. Objects in the center of the visual field are focused on the central part 201 of the retina, while objects on the periphery of the visual field are focused on a peripheral point 203 of the retina. This peripheral point 203 is generally different from the peripheral focus point 202 prior to cataract surgery. In addition, rays entering the eye from the periphery of the visual field are strongly aberrated and the image at peripheral point 203 is out of focus.

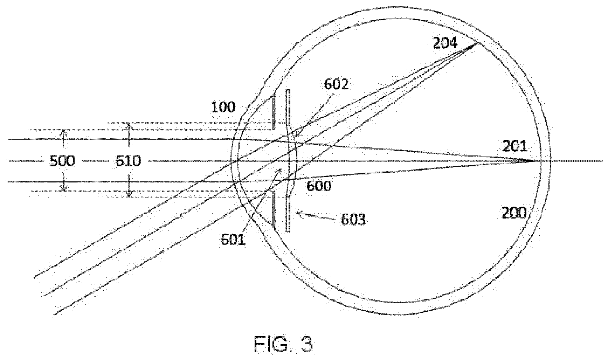

Figure 3 shows an intraocular lens 600 of the invention implanted in the posterior chamber of the eye. An eye is depicted with an improved intraocular lens 600, implanted in the posterior chamber. The light 500 entering through the pupil is focused by the cornea 100 on the retina 200. The intraocular lens 600 has an anterior surface 601, a posterior surface 602, an optical zone 610, and haptics 603. Objects in the center of the visual field are focused on the central part 201 of the retina, while objects in the periphery of the visual field are focused on a peripheral point 204 of the retina. This peripheral point 204 is close to the peripheral focus point 202 prior to cataract surgery. In addition, rays entering the eye from the periphery of the visual field are less aberrated and the image at peripheral point 204 is more focused.

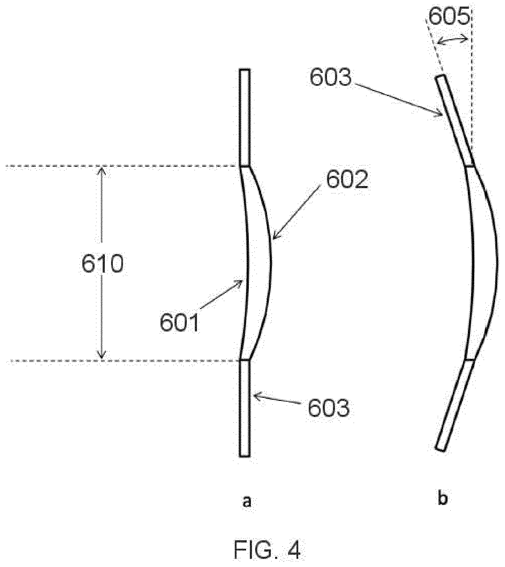

Figures 4a and 4b show two embodiments of intraocular lenses of the invention. Figure 4a shows the intraocular lens 600, with an anterior zone 601 that is concave and a posterior zone 602 that is convex, its optical zone 610, about 6 mm in diameter, and the haptics 603. Figure 4b shows the haptics 603 with a certain angle 605 to a plane passing through the junctions between the central part and the peripheral part. This plane shall be perpendicular to the optical axis of the eye in which the lens is intended to be implanted.

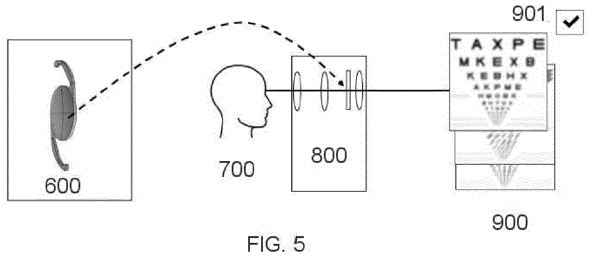

Figure 5 shows an adaptive optics visual simulator 800, which simulates different profiles of intraocular lenses 600 for a patient 700. The patient 700 evaluates the results of the simulations 900 through a visual test at different distances (i.e. far, intermediate, close) and chooses the most optimal result 901 according to his/her visual necessities. This is used to determine the net value of asphericity on the IOL to be implanted.

The present invention refers to the design of a new posterior chamber intraocular lens 600 that simulates the image formation properties of the natural crystalline lens, in the specific referring to the induced field curvature and to the improvement of the visual quality on the far periphery of the retina. Moreover, it can offer extended depth of focus both for central and peripheral vision and therefore reduce the dependence on spectacles postoperatively as well as allow for variability of the shape of peripheral retina. The IOL 600 is manufactured from one of the existing materials suitable of intraocular implantation such as hydrophobic acrylic, hydrophilic acrylic, silicone and can be produced either by machining or by molding.

The intraocular lens 600 has a central part with a diameter between 5 and 7 mm (preferably around 6 mm), with the required optical properties, and a peripheral part that extends to a diameter between approximately 10 and 14 mm (the haptics 603) that help center and stabilize the intraocular lens 600 in the lens capsule once the cataract crystalline lens has been removed. The haptics 603 are selected from a range of designs established in the scientific literature. In the surgical process, the lens 600 is bent and injected into the prepared capsule through a small incision in the limbus or peripheral cornea.

In an embodiment of the intraocular lens 600, it is manufactured by molding acrylic hydrophobic material with a refractive index of approximately 1.53. The anterior surface 601 of the intraocular lens 600 is concave aspherical, and the posterior surface 602 is convex aspherical. The optical part 610 of the lens 600 is circular and has a diameter of approximately 6 mm. The anterior surface 601 has a negative power when submerged in the aqueous humour of the eye (or a similar aqueous solution, such as saline solution), while the posterior surface 602 has a positive power. The combination of these two surfaces produces a refractive power of the order of 20 D when the intraocular lens 600 is immersed in the aqueous humour of the eye (or a similar aqueous solution, such as saline). The radius of curvature of the anterior surface 601 can be changed to produce intraocular lenses 600 of the same type, but with different diopter powers, as required in cataract surgery. The posterior surface 602 has an appropriate conical constant such that the spherical aberration of the lens 600 compensates for the spherical aberration of the average cornea of a human eye. In this embodiment, the total spherical aberration of the eye is practically zero, achieving optimal image quality for central and peripheral vision. The haptics 603 form an angle 605 (see Figure 4b) with respect to a plane passing through the junctions between the central part and the peripheral part and which is perpendicular to the optical axis of the eye in which the lens 600 of approximately 2 degrees is intended to be implanted.

In another embodiment of the present invention, the intraocular lens 600 is manufactured by machining or molding hydrophobic acrylic material with a refractive index of between about 1.46 to about 1.55. The anterior surface 601 of the intraocular lens 600 is concave aspherical and the posterior surface 602 is convex aspherical. The optical part 610 of the lens 600 is circular and has a diameter of approximately 5 to 7 mm. The anterior surface 601 has a negative power when submerged in the aqueous humour of the eye (or a similar aqueous solution, such as saline solution), while the posterior surface 602 has a positive power. The combination of these two surfaces produces a refractive power of the order of 20 D when the intraocular lens 600 is immersed in the aqueous humour of the eye (or a similar aqueous solution, such as saline). The radius of curvature of the anterior surface 601 can be changed to produce intraocular lenses 600 of the same type, but with different diopter powers, as required in cataract surgery. The posterior surface 602 has an appropriate conical constant such that the total power of the intraocular lens 600 remains constant, but that the negative spherical aberration of the lens 600 induces a concrete value equal to or greater (in absolute value) than that of the average cornea of a human eye. In this embodiment, the total spherical aberration of the eye can be adjusted between values close to zero and a negative value approximately 5 times greater (in magnitude) than that of the average cornea of a human eye. In this way, a greater compromise between image quality and depth of focus can be achieved. The optical part 610 has a diameter of approximately 6 mm and the haptics 603 form an angle 605 with respect to a plane that passes through the joints between the central part and the peripheral part and that is perpendicular to the optical axis of the eye in which the lens 600 is to be implanted, which varies between 0 and approximately 10º.

In another embodiment, the intraocular lens 600 is manufactured by machining or molding any material in the hydrophobic acrylic, hydrophilic acrylic, or silicone group that has refractive indices typically between approximately 1.46 and approximately 1.55. The anterior surface 601 of the intraocular lens 600 is concave aspherical and the posterior surface 602 is aspherical convex. The optical part 610 of the lens 600 is circular and has a diameter ranging from approximately 5 to 7 mm. The anterior surface 601 has a negative power when submerged in the aqueous humour of the eye (or a similar aqueous solution, such as saline solution), while the posterior surface 602 has a positive power. The combination of these two surfaces produces a refractive power of the order of 20 D when the intraocular lens 600 is immersed in the aqueous humour of the eye (or a similar aqueous solution, such as saline). The radius of curvature of the anterior surface 601 can be changed to produce intraocular lenses 600 of the same type, but with different diopter powers, as required in cataract surgery. The balance of diopter power between the anterior 601 and posterior 602 surfaces can be changed to compensate for total or partial peripheral astigmatism in the eye. Both surfaces, anterior 601 and posterior 602, may have conical constants defined in such a way that the power of the intraocular lens 600 remains almost constant but the negative spherical aberration of the lens 600 has a particular value equal to or greater (in absolute value) than that of the average human cornea. In this embodiment, the total spherical aberration in the eye can be adjusted from practically zero to a value approximately 5 times greater than the spherical aberration value of the normal eye (in absolute value). In this way, the best compromise between image quality and depth of focus is achieved. The optical zone 601 has a diameter of approximately 6 mm and the haptics 603 form an angle 605 with respect to a plane passing through the joints between the central part and the peripheral part and which is perpendicular to the optical axis of the eye in which the lens 600 is to be implanted, which varies between approximately 0 and approximately 10 degrees. The angulation 605 of the 603 haptics 603 controls the axial depth at which the intraocular lens 600 is implanted into the lens capsule. This adjustment, combined with the power balance between the anterior surface 601 and the posterior surface 602 can be varied to control the shape of the field curvature in the eye and make it match the shape of the retina. This adjustment helps correct peripheral refractive errors in the eye, a task that may be of particular importance for the control of myopia development following congenital cataract surgery. In a similar embodiment, the optical part 610 of the lens 600 can be oval. The longest axis of the oval would be implanted horizontally to reduce the phenomena associated with light diffusion and glare produced by the edge of the lens 600 in the distant temporal field of vision.

In another embodiment, the lens 600 is manufactured with one of the materials mentioned above, the anterior surface 601 of the lens 600 being concave aspherical and the posterior surface 602 being convex aspherical. The optical part 610 of the lens 600 is circular and has a diameter between approximately 5 and 7 mm. The central and peripheral refraction of a patient 700 is measured, as well as the topography of his cornea and the central and peripheral biometries. Corneal topography information, the axial placement provided for the intraocular lens 600 during implantation, as well as central and peripheral biometries are used to select from a range the angulation 605 of the haptics 603 and the balance between the anterior 601 and posterior 602 surface curvature radii to induce a desired central and peripheral post operative refraction.

In another embodiment, the lens 600 is manufactured with one of the materials mentioned above, the anterior surface 601 of the lens 600 being concave aspherical and the posterior surface 602 being convex aspherical. The optical part 610 of the lens 600 is circular and has a diameter of between approximately 5 mm to approximately 7 mm. The vision of patient 700 can be evaluated thanks to an adaptive optics simulator 800, in which a specific amount of spherical aberration (typically negative) is determined to optimize each patient individually. The optimization aims at maximizing the depth of focus without significantly sacrificing the quality of distant vision. When this value is determined, the patient's corneal topography and biometry are used to select from a range of available intraocular lenses 600 that have different conical constants on at least one of the surfaces 601 or 602, to provide adequate post-operative refraction and depth of field.

A method for optimizing depth of focus and the image quality in the periphery of the visual field uses intraocular lenses 600 of those described above and comprises the following steps (Figure 5):
- to provide a series of intraocular lenses 600 of different net value of asphericity,
- to place a patient 700 in front of a visual simulator 800 with adaptive optics, which emulates different intraocular lens profiles with different net value of asphericity,
- realization of different simulations 900 with different intraocular lens profiles through a visual test at different distances,
- selection of the optimal result 901 of the visual test, and determination of the net value of asphericity of the intraocular lens, and
- choice of the intraocular lens 600 presenting the determined net value of asphericity, for implantation in the eye of patient 700.

Another method for optimizing depth of focus and image quality at the periphery of the visual field uses intraocular lenses 600 of those described above and comprises the following steps:
- measurement of eye shape and/or refraction at the periphery, and
- depending on the measurements made, to customize the shape of the intraocular lens 600 and its net value of asphericity, for its implantation in the eye of patient 700.

Although some embodiments of the invention have been described and represented, it is clear that modifications can be made to them within its scope, which should not be considered to be limited to those embodiments, but only to the content of the following claims.

## Claims

1. Intraocular lens (600), comprising a central part and a peripheral part, the central part being the optical part (610) and the peripheral part comprising haptics (603),
the central part comprising:
- an aspherical concave anterior surface (601), which is the surface closest to the iris of the eye once the lens (600) has been implanted in the eye, and
- an aspherical convex posterior surface (602), which is the surface closest to the retina of the eye once the lens (600) has been implanted in the eye,
**characterized in that**:
- the radius of curvature of the posterior surface (602) of the central part is smaller than the radius of curvature of the anterior surface (601) of the central part, with a ratio between radii of between 2 and 6, and
- the haptics (603) are arranged at an angle (605) of between 0° and 10° with respect to a plane passing through the joints between the central part and the peripheral part and which is perpendicular to the optical axis of the eye in which the lens (600) is intended to be implanted.

2. Intraocular lens (600), according to claim 1, in which the haptics (603) are arranged at an angle (605) of 2° with respect to a plane passing through the joints between the central part and the peripheral part and which is perpendicular to the optical axis of the eye in which the lens (600) is intended to be implanted.

3. Intraocular lens (600), according to claim 1 or 2, in which the central part has a circular aspect.

4. Intraocular lens (600), according to claim 3, in which the central part has a diameter of between 5 and 7 mm.

5. Intraocular lens (600), according to claim 1 or 2, in which the central part has an oval aspect.

6. Intraocular lens (600), according to claim 5, in which the long axis of the oval is arranged horizontally once the lens (600) has been implanted in the eye.

7. Intraocular lens (600), according to any of the above claims, which is made of a material selected from the group of hydrophobic acrylics, hydrophilic acrylics or silicones.

8. Method for selecting an intraocular lens to optimize depth of focus and the quality of the image in the periphery of the visual field, which uses intraocular lenses (600) of one of the claims 1 to 7, which comprises the following steps:
- to provide a series of intraocular lenses (600) of different net value of asphericity,
- to place a patient (700) in front of a visual simulator (800) of adaptive optics, which emulates different intraocular lens profiles with different net value of asphericity,
- realization of different simulations (900) with different intraocular lens profiles through a visual test at different distances,
- selection of the optimal result (901) of the visual test, and determination of the net value of asphericity of the intraocular lens, and
- choice of the intraocular lens (600) presenting the determined net value of asphericity for its implantation in the patient's eye (700).

## Patentansprüche

1. Intraokularlinse (600), umfassend einen zentralen Teil und einen peripheren Teil, wobei der zentrale Teil der optische Teil (610) ist und der periphere Teil die Haptik (603) umfasst, wobei der zentrale Teil Folgendes umfasst:
- eine asphärische konkave vordere Fläche (601), dies ist die Fläche, die der Iris des Auges am nächsten ist, sobald die Linse (600) in das Auge implantiert wurde, und
- eine asphärische konvexe hintere Fläche (602), dies ist die Fläche, die der Netzhaut des Auges am nächsten ist, sobald die Linse (600) in das Auge implantiert wurde,
**dadurch gekennzeichnet, dass**:
- der Krümmungsradius der hinteren Fläche (602) des zentralen Teils kleiner ist als der Krümmungsradius der vorderen Fläche (601) des zentralen Teils, wobei das Verhältnis der Radien zwischen 2 und 6 liegt, und
- die Haptik (603) in einem Winkel (605) zwischen 0° und 10° in Bezug auf eine Ebene angeordnet ist, die durch die Gelenke zwischen dem zentralen Teil und dem peripheren Teil verläuft und senkrecht zur optischen Achse des Auges ist, in das die Linse (600) implantiert werden soll.

2. Intraokularlinse (600) nach Anspruch 1, bei der die Haptik (603) in einem Winkel (605) von 2° in Bezug auf eine Ebene angeordnet ist, die durch die Verbindungen zwischen dem zentralen Teil und dem peripheren Teil verläuft und senkrecht zu der optischen Achse des Auges ist, in das die Linse (600) implantiert werden soll.

3. Intraokularlinse (600) nach Anspruch 1 oder 2, bei der der zentrale Teil ein kreisförmiges Aussehen aufweist.

4. Intraokularlinse (600) nach Anspruch 3, bei der der zentrale Teil einen Durchmesser zwischen 5 und 7 mm aufweist.

5. Intraokularlinse (600) nach Anspruch 1 oder 2, bei der der zentrale Teil ein ovales Aussehen aufweist.

6. Intraokularlinse (600) nach Anspruch 5, bei der die Längsachse des Ovals horizontal angeordnet ist, sobald die Linse (600) in das Auge implantiert wurde.

7. Intraokularlinse (600) nach einem der vorhergehenden Ansprüche, die aus einem Material hergestellt ist, das aus der Gruppe von hydrophoben Acrylen, hydrophilen Acrylen oder Silikonen ausgewählt ist.

8. Verfahren zur Auswahl einer Intraokularlinse zur Optimierung der Fokustiefe und der Bildqualität in der Peripherie des Sichtfeldes, das Intraokularlinsen (600) nach einem der Ansprüche 1 bis 7 verwendet, das die folgenden Schritte umfasst:
- Bereitstellen einer Reihe von Intraokularlinsen (600) mit unterschiedlichem Nettowert der Asphärizität,
- Positionieren eines Patienten (700) vor einem visuellen Simulator (800) der adaptiven Optik, der verschiedene Intraokularlinsenprofile mit unterschiedlichem Nettowert der Asphärizität emuliert,
- Durchführen verschiedener Simulationen (900) mit verschiedenen Intraokularlinsenprofilen durch eine visuelle Prüfung in unterschiedlichen Abständen,
- Auswählen des optimalen Ergebnisses (901) des visuellen Tests und Bestimmen des Nettowerts der Asphärizität der Intraokularlinse und
- Auswählen der Intraokularlinse (600), die den bestimmten Nettowert der Asphärizität für ihre Implantation in das Auge des Patienten (700) aufweist.

## Revendications

1. Lentille intraoculaire (600), comprenant une partie centrale et une partie périphérique, la partie centrale étant la partie optique (610) et la partie périphérique comprenant des haptiques (603),
la partie centrale comprenant :
- une surface antérieure concave asphérique (601), qui est la surface la plus proche de l'iris de l'œil une fois que la lentille (600) a été implantée dans l'œil, et
- une surface postérieure convexe asphérique (602), qui est la surface la plus proche de la rétine de l'œil une fois que la lentille (600) a été implantée dans l'œil,
**caractérisée en ce que** :
- le rayon de courbure de la surface postérieure (602) de la partie centrale est inférieur au rayon de courbure de la surface antérieure (601) de la partie centrale, avec un rapport entre les rayons compris entre 2 et 6, et
- les haptiques (603) sont disposées selon un angle (605) compris entre 0° et 10° par rapport à un plan passant par les jointures entre la partie centrale et la partie périphérique et qui est perpendiculaire à l'axe optique de l'œil dans lequel la lentille (600) est destinée à être implantée.

2. Lentille intraoculaire (600) selon la revendication 1, dans laquelle les haptiques (603) sont disposées selon un angle (605) de 2° par rapport à un plan passant par les jointures entre la partie centrale et la partie périphérique et qui est perpendiculaire à l'axe optique de l'œil dans lequel la lentille (600) est destinée à être implantée.

3. Lentille intraoculaire (600) selon la revendication 1 ou 2, dans laquelle la partie centrale a un aspect circulaire.

4. Lentille intraoculaire (600) selon la revendication 3, dans laquelle la partie centrale a un diamètre compris entre 5 et 7 mm.

5. Lentille intraoculaire (600) selon la revendication 1 ou 2, dans laquelle la partie centrale a un aspect ovale.

6. Lentille intraoculaire (600) selon la revendication 5, dans laquelle le grand axe de l'ovale est disposé horizontalement une fois que la lentille (600) a été implantée dans l'œil.

7. Lentille intraoculaire (600) selon l'une quelconque des revendications précédentes, qui est fabriquée à partir d'un matériau choisi dans le groupe des acryliques hydrophobes, des acryliques hydrophiles ou des silicones.

8. Procédé de sélection d'une lentille intraoculaire pour optimiser la profondeur de champ et la qualité de l'image dans la périphérie du champ visuel, qui utilise des lentilles intraoculaires (600) selon l'une des revendications 1 à 7, qui comprend les étapes suivantes :
- fournir une série de lentilles intraoculaires (600) de valeur nette d'asphéricité différente,
- placer un patient (700) devant un simulateur visuel (800) d'optique adaptative, qui émule différents profils de lentilles intraoculaires avec une valeur nette d'asphéricité différente,
- réaliser différentes simulations (900) avec différents profils de lentilles intraoculaires par un test visuel à différentes distances,
- sélectionner le résultat optimal (901) du test visuel et déterminer la valeur nette d'asphéricité de la lentille intraoculaire, et
- choisir la lentille intraoculaire (600) présentant la valeur nette d'asphéricité déterminée pour son implantation dans l'œil du patient (700).
